Europäisches Patentamt

European Patent Office

Office européen des brevets

(11) Publication number: **0 137 749**
**A2**

# EUROPEAN PATENT APPLICATION

(21) Application number: **84306265.4**

(22) Date of filing: **13.09.84**

(51) Int. Cl.⁴: **C 07 C 29/80**
C 07 C 31/08, C 07 C 67/08
C 07 C 69/14, C 07 C 51/48

(30) Priority: **14.09.83 US 532124**

(43) Date of publication of application:
**17.04.85 Bulletin 85/16**

(84) Designated Contracting States:
**BE DE FR GB IT NL**

(71) Applicant: **THE HALCON SD GROUP, INC.**
**2 Park Avenue**
**New York, N.Y. 10016(US)**

(72) Inventor: **Stein, Theodore W.**
**30 Branford Road**
**Hastings-on-the-Hudson New York(US)**

(74) Representative: **Cropp, John Anthony David et al,**
**MATHYS & SQUIRE 10 Fleet Street**
**London, EC4Y 1AY(GB)**

(54) Recovering ethanol from aqueous acetic acid solutions.

(57) Dilute aqueous acetic acid streams are treated by a sequence of steps whereby the acetic acid values are converted to ethanol which may be recovered in substantially anhydrous form. The acetic acid is extracted from water with an extractive solvent and esterified by reaction with an alcohol such as amyl alcohol. The ester is reacted with hydrogen in a hydrogenolysis reaction to form ethanol and the esterification alcohol, and ethanol product is recovered.

./...

FIG. 3

RECOVERING ETHANOL FROM AQUEOUS ACETIC ACID SOLUTIONS
## Prior Art

The present invention relates to the treatment of dilute aqueous acetic acid solutions and particularly to the production and recovery of ethanol, preferably substantially anhydrous ethanol, from dilute aqueous acetic acid solutions.

Dilute aqueous acetic acid solutions are formed in a number of different technologies. For example, great emphasis has been given to the formation of dilute aqueous acetic acid solutions by the biological conversion of waste materials such as sewage, garbage, paper processing wastes, and the like as well as by fermentation of conventional feedstocks such as cornmeal, sugar beets, and the like. See, for example, U. S. Patent 4,377,638 and patents referred to therein. Also such dilute solutions often result from various chemical processing technologies, often as waste streams which pose substantial disposal problems.

A great deal of effort has been directed to devising methods for the recovery of acetic acid from such dilute aqueous solutions. Generally, straightforward distillation procedures, even those employing entraining or azeotroping agents have proved too costly in energy requirements to be commercially acceptable. A substantial amount of effort has been devoted to extraction procedures in order to provide a primary separation between acetic acid and water. For example, U. S. Patent 2,395,010 is illustrative of such prior efforts and describes extraction of acetic acid from water using amyl acetate as a suitable extraction agent followed by a distillation to recover acetic acid product.

U. S. Patents 3,980,701, 3,980,702, 3,980,703, 3,980,704 and 3,997,599 are also illustrative of the efforts of prior workers and describe extraction of acetic acid from dilute aqueous solutions using dialkyl allyl phosphonates, alkyl dialkyl phosphinates, trialkyl phosphine oxides, dialkyl alicyclic amidophosphates, dialkyl sulfoxides and tetralkyl ureas as extraction agents, preferably in combination with organic diluents such as kerosene, carbon tetrachloride, amyl acetate, toluene, ethyl benzyl ketone, dibutyl ether, benzonitrile, 2,2-dichloro propane, heptane, l-nitro-

butane triethyl phosphite, l-pentanol, ethoxy (triethyl) silans and l-fluoropentane. U. S. Patent 2,275,862 shows similar extractions using higher dialkyl ketone extractants while U. S. Patent 2,446,231 employs ethyl disulfide as extractant. Generally, in such procedures, the extracted acetic acid is separated from the solvent by stripping with water or by reaction with ammonia or an aqueous base such as sodium hydroxide.

Even in the extractive procedures the acetic acid is recovered in concentrated form, e.g., as glacial acetic acid, by methods involving extensive distillation. See, for example, "Economics of Recovering Acetic Acid" by W. V. Brown, Chemical Eng. Progress, Vo. 59, No. 12, p. 65-68, (1963) and "Removing Carboxylic Acids from Aqueous Wastes" by R.W. Hensel, Chem. Eng. Progress, P. 55-59, (May 1977). Distillation costs have been very high and these costs have effectively prevented widespread adoption of procedures for recovering valuable and concentrated chemicals from dilute aqueous acetic acid streams.

Methods have long been known for esterifying acids such as acetic acid with various alcohols. Generally such procedures involve the use of acidic type catalysts such as sulfonic acid, organic sulfonic acids, and especially cationic exchange resins having sulfonic acid groups. Because water is a product of the esterification reaction, water must be removed in order to drive the esterification to completion. See, for example, "Encyclopedia of Chemical Technology", Kirk-Othmer, 3rd Edition, Vol. 9, p. 291-300 (1980).

Further it has long been known in the art to convert carboxylic acid esters to the corresponding alcohols by hydrogenolysis techniques. Early patents illustrating such procedures are U. S. Patents 2,079,414 and 2,091,800.

Now, in accordance with the present invention, a process is provided for the treatment of dilute aqueous acetic acid solutions which involves the novel and unique combination of extraction, esterification and hydrogenolysis steps whereby the acetic acid values are recovered in the form of concentrated ethanol. Unlike the efforts of prior workers,while focused on the recovery of acetic acid as such from dilute solutions, the present process avoids many of the prior disadvantages by the unique combination of steps herein set forth.

## Summary of the Invention

The present invention provides a novel and improved process for treating dilute aqueous acetic acid streams such as those which result from biological waste treatment. In accordance with the invention, the dilute aqueous acetic acid stream is contacted with an extraction agent such as amyl acetate or trialkyl phosphine oxide by procedures such as those taught in the prior art whereby the acetic acid is separated from the great predominance of water. The present invention departs from prior art in that instead of recovering acetic acid from the extraction solution by distillation or stripping procedures as practised in the art, the extracted acetic acid is chemically converted first to an acetate ester by reaction with an alcohol and subsequently to ethanol by hydrogenolysis of the said acetate ester. It is then relatively simple and inexpensive to separate the ethanol from the esterification alcohol, which is regenerated during the hydrogenolysis, at a fraction of the distillation costs associated with prior art acetic acid recovery procedures.

The invention will now be described with reference to certain preferred embodiments and with the aid of the accompanying drawings in which

Figure 1 is a schematic flow diagram of an embodiment whereby amyl acetate preferably together with amyl alcohol is the extracting agent and amyl alcohol is the esterifying alcohol;

Figure 2 is a schematic flow diagram of an embodiment of the invention wherein an extractive solvent such as trioctyl phosphine oxide and kerosene is employed together with a separate esterifying alcohol to react with the extracted acetic acid; and

Figure 3 is a simplified schematic flow diagram of the embodiment of Figure 2.

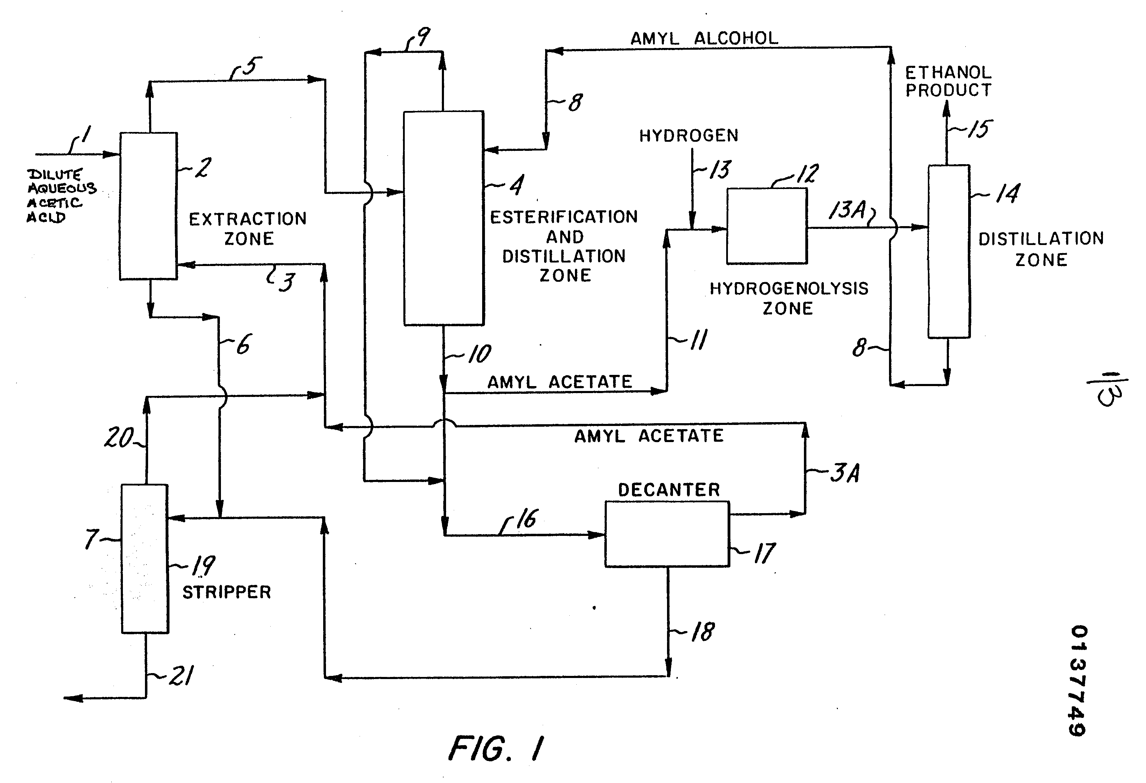

Referring to Figure 1, a dilute aqueous acetic acid stream, illustratively from a biological waste treatment plant, is introduced via line 1 into extraction zone 2. The compositions of such dilute streams normally comprise concentrations by weight of acetic acid in the range 0.1 to 10% acetic acid with the remainder essentially comprising water.

Amyl acetate extracting agent is introduced into zone 2 by means of line 3. Usually the amyl acetate has associated with it minor quantities of amyl alcohol, water, acetic acid and preferably esterification catalyst such as an organic sulfonic acid.

The extraction contact in zone 2 is carried out under conditions known in the art and results in the separation of acetic acid from the vast predominance of water. The extract stream comprising the bulk of the amyl acetate and acetic acid together with amyl alcohol, the esterification catalyst, and minor amounts of water is removed from zone 2 and passed to esterification and distillation zone 4 by means of line 5. The aqueous phase comprising a predominance of the water together with minor quantities of acetic acid, amyl alcohol and amyl acetate passes from extraction zone 2 via line 6 to stripping zone 7.

In zone 4, acetic acid is esterified by catalytic reaction with amyl alcohol with formation of amyl acetate. This esterification reaction may have taken place to some extent in extractor 2 and is substantially completed in zone 4. Amyl alcohol necessary for the esterification is introduced into zone 4 via line 8. Zone 4 is also a distillation zone, heat necessary for the distillation being provided by indirect reboiler means which are not shown.

Zone 4 is most suitably a tower esterifier with amyl alcohol being introduced near the top. The tower esterifier is operated under conditions effective to accomplish substantially complete esterification with water contained in the feed as well as that formed during esterification being distilled overhead and the amyl acetate/amyl alcohol removed as liquid bottoms.

In zone 4 the lower boiling materials mainly comprising water from zone 2 as well as water formed in the esterification reaction, some amyl alcohol and very small amounts of acetic acid and amyl acetate are distilled as overhead and removed from zone 4 by means of line 9.

- 5 -

0137749

The bottoms fraction mainly comprises amyl acetate from zone 2 and amyl acetate from the esterification together with small amounts of amyl alcohol, water, esterification catalyst and acetic acid, and this fraction passes from zone 4 by means of line 10.

This bottoms fraction is divided into two streams. One stream corresponding in amount essentially to the quantity of acetic acid introduced via line 1 passes via line 11 to hydrogenolysis zone 12 wherein it reacts with hydrogen introduced via line 13 to form ethanol and amyl alcohol. Conditions employed are of a generally known type as set forth, for example, in U. S. Patent 2,079,414.

The product from zone 12 passes via line 13A to distillation zone 14 wherein the ethanol and amyl alcohol are separated by conventional distillation procedures. Ethanol product is removed overhead via line 15 and amyl alcohol bottoms passes via line 9 back to zone 4 for further use in esterification.

The separation of the original acetic acid values, now in the form of ethanol, is readily accomplished by distillation from amyl alcohol. An outstanding advantage of the present invention is that this separation can be accomplished in relatively simple equipment employing only a very small fraction of energy needed to carry out the much more difficult distillation of acetic acid from water or acetic acid from amyl acetate, and that product ethanol can be recovered in substantially anhydrous form.

The remaining portion of the bottoms fraction from zone 4 passes via line 16 in combination with condensed overhead from zone 4 to decanter 17. Phase separation takes place in decanter 17 and the organic amyl acetate phase passes via lines 3A and 3 back to extraction zone 2 for further extraction duty.

The aqueous phase from decanter 17 comprises mainly water with small amounts of acetic acid and possibly amyl acetate and amyl alcohol. This aqueous phase passes via line 18 to stripper 19 wherein, together with the aqueous raffinate phase from extraction zone 2 via line 6, it is stripped to separate the bulk of the organic

1254

materials from the water. The organics, mainly amyl acetate, pass overhead via lines 20 and 3 to extraction zone 2 for reuse while the bottoms aqueous phase containing small amounts of acetic acid are removed via line 21 and can be discarded, suitably after neutralization or other treatment.

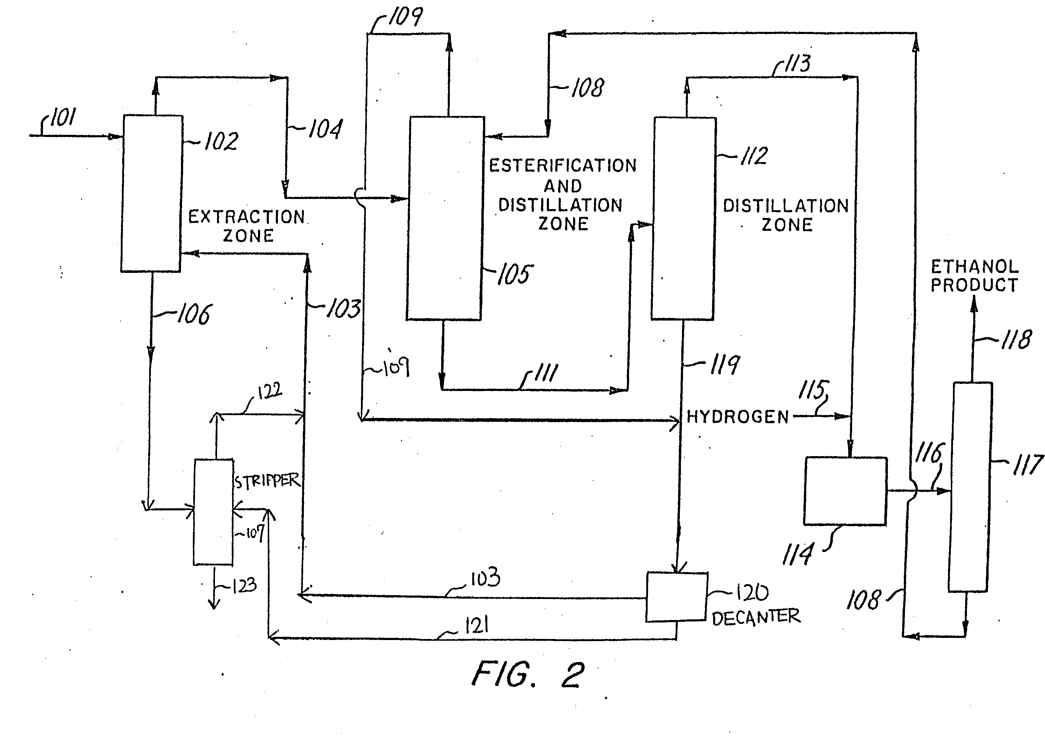

Figure 2 illustrates an advantageous alternative practice of the invention. Referring to Figure 2, the dilute aqueous acetic acid stream is introduced via line 101 to extraction zone 102. The extractive solvent, which is about 1 weight percent trioctyl phosphine oxide in kerosene, together with sulfonic acid esterification catalyst and amyl alcohol is introduced into zone 102 via line 103.

In zone 102 acetic acid is efficiently extracted from water by contact with the extraction solvent. An advantage of this embodiment over that described in connection with Figure 1 is that much less extractive solvent can be used because of the greater extraction efficiency and thus equipment sizes can be substantially reduced.

Extracted acetic acid together with solvent, catalyst, amyl alcohol and small amounts of water pass via line 104 to tower esterifier 105. The aqueous phase from zone 102 passes by means of line 106 to stripper 107.

Additional amyl alcohol is added to tower esterifier 105 by means of line 108. The tower esterifier is operated under conditions such that essentially complete conversion of extracted acetic acid to amyl acetate takes place. Since, in this embodiment the extractive solvent is not an ester, the substantially complete esterification of the extracted acetic acid is facilitated.

Water which had been introduced via line 104 as well as that formed during esterification is removed overhead together with small amounts of organic materials and passed via line 109 to decanter 120. Solvent and amyl acetate, together with some acetic acid, amyl alcohol and esterification catalyst pass as bottoms from esterifier 105 via line 111 and are distilled in zone 112 to separate amyl acetate overhead from the other components of the mixture.

The amyl acetate is passed via line 113 to hydrogenolysis zone 114 wherein it is reacted with hydrogen introduced via line 115 to form ethanol and amyl alcohol. The
hydrogenolysis mixture passes via line 116 to distillation
zone 117 wherein the components are readily separated by
conventional distillation. Product ethanol is recovered via
line 118 while amyl alcohol is recycled to zone 105 by means
of line 108.

The bottoms stream from distillation zone 112 passes
via line 119 where it is combined with esterifier overhead
from line 109 and decanted in decanter 120. The organic phase
comprising trioctyl phosphine oxide in kerosene, esterification catalyst and some amyl alcohol is sent to extraction
zone 102 by means of line 103.

The aqueous phase passes from decanter 120 via line
121 to stripper 107. In stripper 107 organics are stripped
overhead and pass via lines 122 and 103 to extraction zone
102. Bottoms from stripper 107 are purged from the system
through line 123.

Various other extractive procedures which are known
in the art can be employed in carrying out the invention
provided that the extracted acetic acid can be readily
esterified without undue difficulties.

As far as the esterifying alcohol is concerned,
while amyl alcohol is a preferred alcohol other alcohols
which readily esterify with acetic acid and which are readily
separated from ethanol can also be used. Examples of such
alcohols include n-butanol, isobutanol, cyclohexanol, octyl
alcohol, nonyl alcohol, and the like.

The following examples illustrate the invention:

### Example 1

This example is illustrative of practice of the
invention as described in accompanying Figure 1.

Referring to Figure 1, a dilute aqueous acetic acid
stream is fed at the rate of 5257 mols per hour to extraction
zone 2. This acetic acid stream comprises 67 mols per hour
acetic acid together with 5190 mols per hour of water.
Extraction zone 2 is a conventional extraction apparatus
specifically designed for the intimate contact of liquid

1254

streams and the separation of immiscible liquid phases.

The extracting solvent mixture comprising mainly amyl acetate is introduced into extraction zone 2 through line 3. The solvent stream comprises 27 mols per hour water, 2303 mols per hour amyl acetate, 7.4 mols per hour acetic acid, and 41.2 mols per hour amyl alcohol. This stream also contains a catalytically effective amount of a suitable esterification catalyst such as toluene sulfonic acid. In zone 2 the liquid streams are intimately contacted and admixed at a temperature of about 30°C.

Exiting from zone 2 via line 5 is the solvent extract phase which passes to tower esterification zone 4. The solvent extract stream contains 27 mols per hour water, 2300 mols per hour amyl acetate, 63 mols per hour acetic acid, and 41 mols per hour amyl alcohol and also contains the toluene sulfonic acid esterification catalyst. A solvent lean phase is removed from extraction zone 2 by means of line 6 and is passed to stripper 19. This solvent lean phase contains 5190 mols per hour water, 3 mols per hour amyl acetate, 11.4 mols per hour acetic acid, and 0.2 mols per hour amyl alcohol and also contains a trace of the esterification catalyst.

Tower esterification zone 4 is a distillation column designed for accomplishing both distillation separation and also providing liquid contact and sufficient residence time for esterification reaction between the acetic acid and amyl alcohol which is contained in the solvent extract stream introduced via line 5. In zone 4, heat to accomplish the distillation and the esterification is provided by indirect heat exchange through a reboil (not shown). Conditions in the tower esterifier are maintained such that the overhead exits at a temperature of 98°C. and a pressure of about 16 psia absolute. The overhead stream which is removed by means of line 9 contains essentially all of the water produced through the esterification reaction as well as that water which was contained in the feed to zone 4. The overhead vapor stream comprises 81 mols per hour water, about 1.5 mols per hour amyl acetate, about 1.5 mols per hour acetic acid and 18 mols per hour amyl alcohol. This stream is condensed (not shown) by indirect heat exchange and passes via lines 9 and 16 to decanter 17. Amyl alcohol for esterification is introduced into zone 4 by means of line 8. The amyl alcohol stream comprises a trace of water and acetic acid together with about 53.8 mols per hour amyl alcohol.

The bottoms ester stream is removed from zone 4 at a temperature of $160^{O}$C. via line 10 and comprises the esterification catalyst, about 1 mol per hour water, 2352 mols per hour amyl acetate, 8 mols per hour acetic acid, and 23.3 mols per hour amyl alcohol.

The liquid bottoms stream is split with one portion containing effectively the acetic acid introduced into zone 4 now in esterified form and is passed via line 11 into hydrogenolysis zone 12. This amyl acetate stream contains a trace of water, 53.5 mols amyl acetate, 0.15 mols acetic acid and 0.3 mols acetic acid and 0.3 mols amyl alcohol.

Hydrogen is introduced into hydrogenolysis zone 12 via line 13 and in zone 12 the amyl acetate is subjected to a catalytic hydrogenolysis reaction under generally known conditions in order to convert the amyl acetate to amyl alcohol and ethanol. Suitably 107 mols per hour of hydrogen are added via line 13 and the hydrogenolysis is carried out at a temperature of $200^{O}$C. and a pressure of 900 psig using a promoted copper chromite catalyst.

The effluent from zone 12 comprising a trace of water, 0.15 mol per hour acetic acid, 53.8 mols amyl alcohol and 53.5 mols ethanol passes via line 13A to distillation zone 14 wherein by conventional distillation procedures ethanol product is separated as an overhead stream in concentrated and highly pure form. This ethanol product comprising 53 mols ethanol per hour is removed via line 15. The bottoms amyl alcohol passes from zone 14 via line 8 and is returned to zone 4 for further esterification service.

The remainder of the bottoms from zone 4 passes via lines 10 and 16 to decanter 17 wherein it is combined with the condensed overhead from zone 4 and decanted. The upper organic layer is removed via line 3A and is returned to extract zone 2. This stream contains the esterification

1254

catalyst, 27 mols per hour water, 2300 mols per hour amyl acetate, 7.4 mols per hour acetic acid and 41 mols per hour amyl alcohol.

The aqueous phase from decanter 17 passes via line 18 to stripper 19 wherein it is combined with the aqueous phase from extraction zone 2 and stripped in stripper 19. An overhead organics containing stream is removed at $105^{O}$C. and 17 psi$^{a}$ from stripper 19 via line 20 and comprises 2.97 mols per hour amyl acetate and 0.2 mols per hour amyl alcohol. This overhead is condensed (not shown) and passed via lines 20 and 3 and extract zone 2.

The bottoms from stripper 19 is removed via line 21 and comprises 5245 mols per hour water, a trace of amyl acetate, 13.4 mols per hour acetic acid and a trace of amyl alcohol. This stream is suitably discarded after appropriate chemical treatment, if necessary.

From the above it can be seen that the embodiment of the invention described in Figure 2 provides a novel and extremely efficient method for recovering chemical values from dilute aqueous acetic acid streams. The great predominance of the acetic acid is efficiently converted by means of esterification in hydrolysis procedures to ethanol at significantly reduced costs when contrasted to the expense separating acetic acid as such from the aqueous streams by distillation procedures. Ethanol is in its own right a chemical of great value so the production of ethanol by these procedures, taking into consideration the substantially reduced energy requirements, is extremely advantageous.

### Example 2

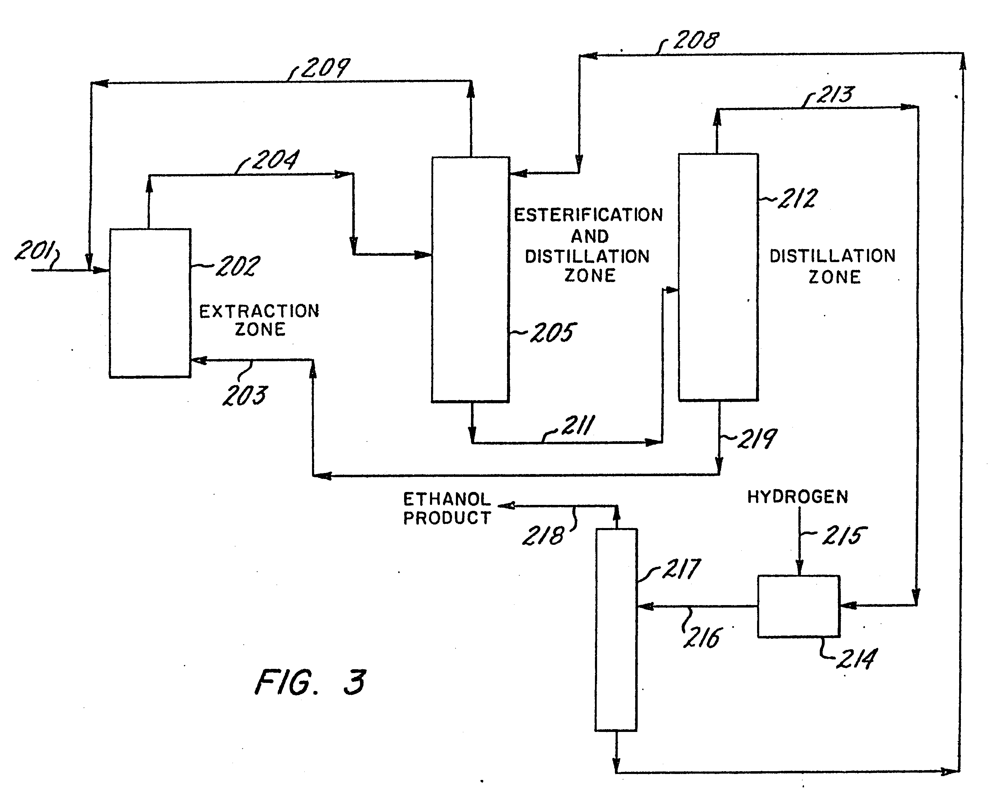

This example illustrates practice of the invention as set forth in accompanying Figure 3. The embodiment of the invention illustrated in Figure 3 involves the use of the combination of trioctyl phosphine oxide in kerosene as the extraction agent.

Referring to Figure 3, a dilute aqueous acetic acid stream comprised of 5496 mols per hour water and 51 mols per hour acetic acid is introduced through line 201 to

1254

extraction zone 202. Like the extraction zone in Figure 1, zone 202 is a conventional apparatus adapted for intimate liquid contact and the separation of immiscible liquid streams. The solvent mixture is introduced by means of line 203 to zone 202 and contains 663 mols per hour of a solvent mixture of 80 mol percent kerosene and 20 mol percent tri-octyl phosphine oxide. The solvent extraction stream is removed from zone 202 by means of line 204 and passes to tower esterification and distillation zone 205. The extraction stream contains 62 mols per hour water, 46 mols per hour acetic acid, a trace of hexanol and essentially all of the kerosene and tri-octyl phosphine oxide.

N-hexyl alcohol is the esterification agent and is introduced into zone 205 through line 208, the hexyl alcohol stream comprising 2 mols per hour acetic acid and 50 mols per hour hexyl alcohol. Conditions in the tower esterification zone 205 are maintained such that essentially all of the water introduced through line 204 and produced by esterification in zone 205 is removed overhead. The overhead stream is removed via line 209 at 99°C. and 16 psia and after condensation (not shown) is decanted (not shown) with the aqueous phase passing via line 209 and 201 back to zone 202. The organic phase from the decantation is returned to the top of tower esterifier 205 (not shown). The bottoms stream essentially containing product ester and solvent is removed from zone 205 by means of line 211 and comprises 44 mols per hour N-hexyl acetate, 2 mols per hour acetic acid, 6 mols per hour N-hexanol and 663 mols per hour of kerosene plus trioctyl phosphine oxide. This stream passes via line 211 to distillation zone 212 wherein the solvent is separated as a bottoms stream and after appropriate cooling (not shown) is passed via lines 219 and 203 to extraction zone 202.

The overhead stream from distillation zone 212 exits at about 110°C. and 17 psia via line 213 and passes to hydrogenolysis zone 214. The overhead stream comprises 44 mols per hour hexyl acetate, 2 mols per hour acetic acid and 6 mols per hour hexyl alcohol. 88 mols per hour hydrogen are introduced via line 215 into zone 214 wherein the hexyl acetate and hydrogen are subjected to a hydrogenolysis reaction at 200°C. and 900 psig using a promoted copper chromite hydrogenation catalyst. The product mixture is removed from zone 214 via line 216 and comprises 2 mols per hour acetic acid, 60 mols per hour hydrogen, 44 mols per hour ethanol and 50 mols per hour N-hexyl alcohol. This mixture passes to distillation zone 217 wherein by conventional distillation procedures ethanol in high purity and in amount of 44 mols per hour is recovered overhead via line 218. The overhead from distillation column 217 is removed at 82.5°C. and 17 psia. The bottoms from distillation zone 217 is removed via line 208 and recycled for further service in the esterification and distillation zone 205.

In order to illustrate the significant economic advantages of the invention, with specific reference to the embodiment illustrated above in Figure 1, the normal distillation requirement to separate acetic acid from amyl acetate by conventional distillation procedures would be about 33 million BTUs per hour whereas the energy requirements involved in the esterification of acetic acid to amyl acetate and removal of amyl acetate in esterification zone 4 is only approximately 5 million BTUs per hour. The enormous saving accomplished in this separation provides significant economic advantages for the sequence of steps which comprise the process of this invention.

1254

- 13 -

CLAIMS:

1. A process for treating dilute aqueous acetic acid which comprises:

   (a) extracting acetic acid from water with an extractive solvent,

   (b) esterifying the said extracted acetic acid by reaction with an alcohol,

   (c) reacting the esterified extracted acetic acid with hydrogen to form ethanol and an esterification alcohol, and

   (d) separating said ethanol from said esterification alcohol.

2. A method as claimed in claim 1 wherein the extractive solvent is amyl acetate.

3. A method as claimed in claim 1 wherein the extractive solvent is a solution of trioctyl phosphine oxide in kerosene.

4. A method as claimed in any one of claims 1 to 3 wherein the extracted acetic acid is esterified by reaction with amyl alcohol.

FIG. 1

DILUTE AQUEOUS ACETIC ACID 1

2 EXTRACTION ZONE

5

9

4 ESTERIFICATION AND DISTILLATION ZONE

AMYL ALCOHOL

8

ETHANOL PRODUCT 15

HYDROGEN 13

12

13A

14 DISTILLATION ZONE

HYDROGENOLYSIS ZONE

3

6

11

10 AMYL ACETATE

8

20

AMYL ACETATE

3A

DECANTER

16

17

7

19 STRIPPER

18

21

1/3

0137749

FIG. 2

2/3

0137749

FIG. 3